# EUROPÄISCHE PATENTSCHRIFT

(11) **EP 0 721 005 B1**
(45) Veröffentlichungstag und Bekanntmachung des Hinweises auf die Patenterteilung: **16.06.1999**
(21) Anmeldenummer: 95119850.6
(22) Anmeldetag: 15.12.1995
(51) Int. Cl.: C09D 17/00, C09D 11/02, C09D 5/36

(54) **Pigmentzubereitung und deren Verwendung**
Pigment composition and use
Composition pigmentaire et son utilisation

(30) Priorität: 27.12.1994 DE 4446456
(43) Veröffentlichungstag der Anmeldung: 10.07.1996
(73) Patentinhaber: MERCK PATENT GmbH, 64293 Darmstadt (DE)
(72) Erfinder: Heinz, Dieter, D-64646 Heppenheim (DE); Mohr, Heinz, D-67346 Speyer (DE); Weitzel, Joachim, D-64293 Darmstadt (DE)

(56) Entgegenhaltungen:
- US-A- 4 681 632
- DATABASE WPI Week 9139 Derwent Publications Ltd., London, GB; AN 91-281861 & CA-A-2 023 118 (HEWLETT PACKARD) , 17.Juli 1991

## Beschreibung

Die vorliegende Erfindung betrifft eine Pigmentzubereitung im wesentlichen bestehend aus ein oder mehreren plättchenförmigen Glanzpigmenten, einem Phosphatderivat und sphärischen Teilchen mit einer Teilchengröße von 0,05 - 150 µm, sowie deren Verwendung, insbesondere in Druckfarben.

Druckfarben bestehen in der Regel aus Bindemittel, Pigmenten oder Farbstoffen und Additiven. Bei Druckerzeugnissen für Verpackungsdrucke, Etiketten und hochwertige Zeitschriften tritt immer häufiger die Forderung auf den abgebildeten Gegenständen einen speziellen Glanz zu verleihen.

Glanzpigmente enthaltende Druckfarben zeigen alle den Nachteil, daß sie Probleme mit der Fortdruckstabilität aufweisen. Sie neigen dazu auf Farbwerk, Druckplatte und Gummituch schnell aufzubauen oder zu sedimentieren, so daß ein problemloser Fortdruck über 10000 Bögen in der Regel nicht möglich ist. Ein grundsätzliches Problem ist die starke Neigung von Glanzpigmenten, insbesondere Perlglanzpigmenten, bedingt durch die plättchenförmige Struktur sowie der speziellen chemisch/physikalischen Oberflächeneigenschaften zur Bildung von Agglomeraten in der Druckfarbe, wobei die Pigmente stapelförmig aufeinanderliegen und aufgrund von starker Adhäsion nur schwer zu separieren sind. Weiterhin ist die Glanzgebung solcher Andrucke in der Regel nicht zufriedenstellend, was auf die unzureichende Menge an übertragenden Pigmenten auf das Druckerzeugnis zurückzuführen ist. Die Farbe verarmt auf dem Transportweg über Farbwerk, Platte und Gummituch an Pigment. Letzteres reichert sich an exponierten Stellen auf Platte und Tuch an und führt zu Aufbauen und Pelzen.

In der Regel kommen nur Glanzpigmente mit einer sehr kleinen mittleren Teilchengröße in Frage, da die Teilchengröße kritisch ist für die Pigmentübertragung beim Druck. Solche Pigmente existieren nur für perlweiße und pastell abgetönte Farbgebungen, nicht aber für die sehr interessanten Gold-, Silber-, Bronze- und Kupfertöne. Bisher konnten im Offset-Verfahren solche Farbtöne mit Glanzpigmenten nicht zufriedenstellend erzeugt werden.

Der Einsatz von plättchenförmigen Wismutoxichloridpigmenten zur Erzielung eines Perlglanzeffektes führt ebenfalls nicht zu befriedigenden Ergebnissen, da die Pigmentteilchen sehr empfindlich gegen mechanische Scherkräfte sind und im Farbspalt zermahlen werden.

Die Verwendung von Perlglanzpigmenten in Druckfarben für den Offsetdruck ist aus der DE 29 03 212 bekannt, wo die Pigmentierung von einem handelsüblichen Öldrucklack mit einem vorzugsweise sehr feinteiligen Perlglanzpigment offenbart wird. Die dort beschriebene Offset-Drucklack-Zubereitung zeichnet sich dadurch aus, daß der Anteil an Perlglanzpigmenten sehr hoch ist, wobei die Obergrenze der Pigmentkonzentration in der Suspension im wesentlichen nur durch die zu fordernde Fließfähigkeit des Gemisches begrenzt ist. Der Anteil an Perlglanzpigmenten liegt dabei im Bereich bis 65 Gew.%. Lacke mit einem derart hohen Pigmentanteil sind sehr viskos und müssen gegebenenfalls mit einem Verdünner fließfähiger gemacht werden um in herkömmlichen Offset-Druckmaschinen verarbeitbar zu sein. Versuche haben gezeigt, daß im Gegensatz zur Lehre aus der DE 29 03 212, bei solch hohen Pigmentierungen die Pigmentübertragung vom Farbwerk auf das Substrat gestört ist. Es treten Fortdruckstörungen wie Aufbauen und Pelzen auf, d.h., daß der erreichbare Perlglanzeffekt allein durch eine hohe Pigmentierung nicht optimiert werden kann. Weiterhin zeigen die Farbsysteme gemäß der DE 29 03 212 in der Regel eine nicht ausreichende Punktschärfe, so daß die dort beschriebenen Farbsysteme im wesentlichen auf den Druck von Volltonflächen beschränkt sind.

Es bestand daher die Aufgabe eine Pigmentzubereitung, insbesondere für Druckfarben, zu finden, die Glanzpigmente, insbesondere Perlglanzpigmente enthält, die durch eine spezielle Oberflächenpräparation zum einen eine höhere Pigmentierung der Druckfarbe als auch zufriedenstellende Fortdruckeigenschaften erzielt.

Überraschenderweise wurde gefunden, daß die Güte des Glanzeffektes sowie die Menge an übertragenen Pigmentteilchen in einer Druckfarbe optimal wird, wenn man eine Pigmentzubereitung verwendet, die im wesentlichen aus ein oder mehreren Glanzpigmenten, einem Phosphatderivat und sphärischen Teilchen besteht. Die sphärischen Teilchen sollten dabei eine Teilchengröße von 0,05- 150 µm, vorzugsweise 0,05 - 50 µm, und insbesondere 1 - 30 µm aufweisen.

Die Verwendung von sphärischen Teilchen in Offset-Druckfarben ist bereits aus der JP 62-143 984 bekannt. Die dort beschriebenen sphärischen Partikel aus Harz oder Wachs mit einem Durchmesser von 0,1 - 30 µm werden in Mengen von 0,1 - 20 Gew.% in die Druckfarbe, die keinerlei Glanzpigmente enthält, eingerührt, um die Aufbaueigenschaften zu verbessern und das Rupfen des Papiers vom Gummituch zu verhindern.

Bei der erfindungsgemäßen Pigmentzubereitung wird durch die sphärischen Teilchen verhindert, daß die plättchenförmigen Glanzpigmente sich in nennenswertem Umfang aufeinanderlegen und damit eine starke Adhäsion ausüben können. Wie elektronenmikroskopische Aufnahmen zeigen, lagern sich die sphärischen Teilchen an der Oberfläche des plättchenförmigen Glanzpigmentes an und ermöglichen daher wie eine Art Kugellager die leichte Verschiebbarkeit der Pigmentplättchen in der Druckfarbe gegeneinander, wodurch ein Aufbauen und Pelzen der Pigmente während des Druckvorganges weitgehendst unterbunden wird.

Bei der Verwendung der erfindungsgemäßen Pigmentpräparation in Druckfarben können die sphärischen Teilchen schon bestehende Pigmentagglomerate "zerschlagen", indem die sphärischen Teilchen beim Druckvorgang in die Pigmenthäufungen gepreßt werden und diese Verbunde "sprengen". Die größeren sphärischen Teilchen können zum Teil dabei zerstört werden. Im Druckerzeugnis findet man vorzugsweise nur noch Teilchen mit einem kleineren Teilchendurchmesser, während die Bruchstücke lockernd im Pigmentverbund wirken.

Gegenstand der Erfindung ist somit eine Pigmentzubereitung im wesentlichen bestehend a.us ein oder mehreren Glanzpigmenten, einem Phosphatderivat und sphärischen Teilchen mit einer Teilchengröße von 0,05 - 150 µm.

Geeignete sphärische Materialien sind insbesondere Glas-, Wachs- oder Polymerhohlkugeln aus Vinylharzen, Nylon, Silicon, Epoxyharzen, Olefinharzen, Polystyrolen sowie anorganische Materialien, wie z.B. TiO₂, SiO₂ oder ZrO₂. Vorzugsweise werden Hohlkugeln, ferner auch Vollkugeln, mit einer Teilchengröße von 0,05 bis 150 µm verwendet. Insbesondere bevorzugt werden in der erfindungsgemäßen Pigmentzubereitung Glas-, Wachs- oder Polymerhohlkugeln eingesetzt.

Sphärische Teilchen auf Basis von SiO₂ in einem Teilchenbereich von 3- 10 µm sind z.B. als Materialien für die Hochdruckflüssigkeitschromatographie bekannt und werden z.B. als LiChrospher von der Fa. Merck, Darmstadt vertrieben. Vorzugsweise werden solche Materialien in monodisperser Form, d.h. mit einer möglichst einheitlichen Teilchengröße, eingesetzt. Bekannt sind solche monodispersen sphärischen Teilchen auf Basis von SiO₂, TiO₂ und ZrO₂. Monodisperses SiO₂ kann beispielsweise nach der DE 36 16 133 hergestellt werden. Glashohlkugeln werden beispielsweise unter dem Handelsnamen Q-CEL von der Fa. PQ Corporation, USA oder Scotchlite von der Fa. 3M, Frankfurt, BRD vertrieben.

Die verbesserte Deagglomeration der Glanzpigmente in einer Druckfarbe zeigt sich bereits mit geringen Mengen an sphärischen Teilchen in der Pigmentzubereitung. So werden bereits bei der Verwendung von Glanzpigmenten mit einem Gehalt von 0,5 Gew.% an sphärischen Teilchen bezogen auf das Trockenpigment deutlich verbesserte Fortdruckeigenschaften bei Druckfarben, die die erfindungsgemäße Pigmentpräparation enthalten, festgestellt. In der Regel werden Glanzpigmente mit einem Gehalt von 1- 10 Gew.%, insbesondere 1 - 5 Gew.% an sphärischen Teilchen in der Pigmentzubereitung verwendet.

Weiterer wichtiger Bestandteil der erfindungsgemäßen Pigmentzubereitung ist das Glanzpigment. Die Pigmentzubereitung kann dabei auch ein Gemisch unterschiedlicher Glanzpigmente enthalten, um z.B. bestimmte Farbgebungen zu erzielen. Der Anteil an Glanzpigmenten in der Pigmentzubereitung kann dabei bis zu 99,4 Gew.% betragen.

Die Herstellung der Pigmentzubereitung ist einfach und leicht zu handhaben. Zunächst wird das Glanzpigment mit den sphärischen Teilchen durch Schütteln oder in einem Trockenmischer intensiv gemischt. Anschließend erfolgt die Zugabe des Phosphatderivats, das entweder als feinkörniges Pulver eingerührt oder in Form einer Suspension bestehend aus Salz und einer flüssigen Komponente zugegeben wird. Als flüssige Komponente sind insbesondere Mineralöle oder andere nicht trocknende Öle, wie z.B. Leinöl geeignet, ferner trocknende Öle, wie z.B. Sojaöl, sowie Wasser oder organische Lösemittel. Die flüssige Komponente sollte dann maximal 10 Gew.% bezogen auf die Pigmentzubereitung betragen. Die Pigmentzubereitung kann auch hergestellt werden, indem man alle Komponenten gleichzeitig vorlegt und dann intensiv miteinander mischt. Die fertige Pigmentzubereitung kann dann Formulierungen, wie.z.B. Druckfarben, Lacke, Farben, Kunststoffe und kosmetischen Zubereitungen zugemischt werden.

Als Glanzpigment werden vorzugsweise handelsübliche Metalleffektpigmente, wie plättchenförmiges Eisenoxid, Aluminiumplättchen, z.B. Standart von der Fa. Eckart, Effektpigmente, wie z.B. Paliochrom® von der BASF, sowie Perlglanzpigmente - mit Metalloxiden beschichtete Glimmerschuppenpigmente - erhältlich z.B von der Fa. Merck, Darmstadt unter dem Handelsnamen Iriodin® verwendet. Letztere sind z.B. bekannt aus den deutschen Patenten und Patentanmeldungen 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 und 32 35 017. Vorzugsweise werden für die Druckfarbe Perlglanzpigmente verwendet. Insbesondere werden mit TiO₂ und/oder Fe₂O₃ beschichtete Glimmerpigmente, SiO₂-Flakes, Glasflakes, Keramikflakes oder synthetische trägerfreie Plättchen eingesetzt.

Zusätzlich zu den Glanzpigmenten und sphärischen Teilchen kann die erfindungsgemäße Pigmentzubereitung auch noch Rußteilchen, Fluoreszenz- und/oder organische Buntpigmente enthalten. Vorzugsweise besteht die Zubereitung dann aus 50 - 100 Gew.% an Glanzpigmenten und 0 - 50 Gew.% an Rußteilchen, Fluoreszenz- und/oder Buntpigmenten. Der Gehalt aller Pigmente in der Pigmentzubereitung sollte dabei aber 99,4 Gew.% nicht überschreiten.

Als weiteren wichtigen Bestandteil enthält die erfindungsgemäße Pigmentzubereitung ein Phosphatderivat in Mengen von 0,1 - 5 Gew.%, vorzugsweise 1 - 3 Gew.%. Geeignete Phosphatderivate sind z.B. die höheren und niedrigeren Metapolyphosphate sowie Pyrophosphate. Besonders bevorzugt sind die Alkalimetapolyphosphate, insbesondere das Natriummetapolyphosphat.

Häufig empfiehlt es sich in die Pigmentzubereitung noch ein Dispergiermittel einzurühren. Alle dem Fachmann bekannten Dispergiermittel können verwendet werden, z.B. wie sie in Karsten, Lackrohstofftabellen, 9. Auflage 1992, beschrieben werden. Insbesondere geeignet sind Dispergiermittel auf Basis von Polyacrylaten oder Polymethacrylaten. Die eingesetzte Menge an Dispergiermittel sollte nicht mehr als 10 Gew.% betragen, vorzugsweise 0,1 - 5 Gew.%.

Die erfindungsgemäße Pigmentzubereitung eignet sich insbesondere zur Pigmentierung von Druckfarben. Derartige pigmentierte Druckfarben können für alle bekannten Drucktechniken, insbesondere für Offsetdruck, Hochdruck, Letterset, Tiefdruck, Flexodruck, Siebdruck, weiterhin für die Überdrucklackierung als auch für Beschichtungen eingesetzt werden. Bevorzugt wird sie für den Offsetdruck verwendet. Dabei kann sie sowohl für den Rollenoffsetdruck als auch den Bogenoffsetdruck im Trocken- und Naßverfahren verwendet werden; insbesondere geeignet ist sie jedoch für den Bogenoffsetdruck im Naßverfahren.

Die Dispergierung der Pigmentpräparation in die Druckfarbe bzw. das Bindemittel erfolgt dann mit Hilfe eines Rührwerks mit Propeller- oder Flügelrührer, gegebenenfalls bei unterschiedlichen Dispergiertemperraturen. Dabei werden die Pigmentteilchen vom Bindemittel umhüllt. Die aus den Geräten auslaufenden Mahlpasten werden anschließend mit den Additiven zum fertigen Produkt versetzt.

Für die Herstellung einer pigmentierten Offset-Druckfarbe können alle handelsüblichen Bindemittel verwendet werden. Derartige Bindemittel bestehen aus bekannten synthetischen oder natürlichen Harzen, gegebenenfalls trocknenden Ölen, Mineralölen und Additiven, wie sie beispielsweise in Karsten, Lackrohstofftabellen, 9. Auflage 1992, beschrieben werden. Vorzugsweise besitzen die verwendeten Harze eine relativ niedrige Schmelz- bzw. Lösemittelviskosität. Aber auch hochpolymerisierte und hochviskose Anteile können enthalten sein. Als besonders geeignet haben sich Kombinationen aus Hartharzen und Alkydharzen erwiesen, da diese die Glanzpigmente besser benetzen und glänzendere sowie reibechtere Drucke ergeben. Insbesondere werden Bindemittel verwendet, die aus 50 - 90 Gew.% Hartharz und 5 - 50 Gew.% Alkydharz zusammengesetzt sind. Bei den verwendeten Hartharzen handelt es sich vorzugsweise um Kohlenwasserstoffharze. Die verwendeten Kohlenwasserstoffharze können eine Säurezahl nahe 0 mg KOH/g Substanz haben, sie können aber auch modifiziert sein und Säurezahlen bis 30 mg KOH/g Substanz aufweisen. Weiterhin kann das Bindemittel 1 - 50 Gew.% eines Mineralöls enthalten. Die Farbanteile sind in einer Weise aufeinander abgestimmt, daß eine für die niedrige Farbviskosität geeignete und stabile Farb/Wasser-Balance erreicht wird.

Die Trocknung der Druckfarbe erfolgt durch oxidative Polymerisation der Harze und durch Öle, wie z. B. Lein-, Holz- oder Rizinusöl, die beim Drucken in das Papier wegschlagen. Der Trocknungsvorgang kann durch Zusätze von Trocknungskatalysatoren (Sikkative), meist fettsaure Salze von Kobalt, Blei, Mangan usw., weiter beschleunigt werden. Der Anteil an trocknenden Ölen in den erfindungsgemäßen Offset-Druckfarben liegt im Bereich von 0 - 50 Gew.%, vorzugsweise bei 0 - 30 Gew.%. Weitere Additive können in die Druckfarbe eingebracht werden, um die Farbeigenschaften für spezielle Anwendungen zu verändern. Diese Zusätze können Wachsverbindungen, Trocknungsmittel, Dispersionsmittel, Lösemittel, Verdicker, Gleitmittel, Pigmentfüllstoffe und/oder Antioxidantien sein. Weitere Details zu den grundsätzlichen Eigenschaften von Offset-Druckfarben finden sich in A. Rosenberg, Der Polygraph (11), 1153 (1987) bzw. B. Grande, Coating (4), 138 (1987).

Häufig ist bei den sehr niedrigviskosen pigmenthaltigen Druckfarben keine ausreichende Punktschärfe gegeben. Diese ist jedoch gerade in feinen Rastern erforderlich um Zusetzen des Drucks zu vermeiden. Es empfiehlt sich daher die Druckfarbe mit Struktur zu versehen. So kann die Zugabe eines Strukturbildners die Punktschärfe ausreichend verbessern. Vorzugsweise enthält die erfindungsgemäße Druckfarbe dann 0,1 - 3 Gew.% eines Strukturbildners. Neben der verbesserten Punktschärfe zeigt eine derartig modifizierte Druckfarbe eine deutlich bessere Pigmentübertragung und bessere Fortdruckeigenschaften.

Durch die Wahl der Harze in den Bindemitteln und den Anteil an Glanzpigmenten in der Druckfarbe können die für den Druckprozeß ausschlaggebenden Parameter wie Dispergierbarkeit, Zügigkeit und Viskosität individuell eingestellt werden. Die Viskosität und die Zügigkeit sind bei gleichem Farbaufbau voneinander abhängig, können aber durch speziellen Farbaufbau auch gezielt einzeln verändert werden. Hierbei ist zu beachten, daß Druckfarben mit einem zu hohen Grad an Zügigkeit verursachen können, daß Teile des Papiers reißen (Rupfen). Farben mit ungenügender Zügigkeit werden nicht in geeigneter Weise bei dem Druckvorgang übertragen. Wenn das Eindringen der Farbe zu groß ist, wird die Farbe auf der entgegengesetzten Seite des Papiers sichtbar oder verursacht eine Fleckigkeit oder Undeutlichkeit der Abbildung. Eine schlecht kontrollierte Eindringung kann Verschmieren und Ablegen verursachen. Im Gegensatz dazu werden zu viskose Farben nicht in geeigneter Weise aus den Füllquellen zu den Walzen fließen. Die auf dem Markt befindlichen Druckfarben sind auf Viskositäten im Bereich 12 - 30 Pa.s eingestellt. Druckfarben enthaltend die erfindungsgemäße Pigmentpräparation können auf Viskositäten < 12 Pa.s, vorzugsweise < 10 Pa.s, insbesondere < Pa.s, eingestellt werden. Die Druckfarbe zeigt dabei keinerlei Probleme mit dem Abspritzen von den Farbwalzwerken, auch nicht bei Druckgeschwindigkeiten von 10000 Bögen pro Stunde.

Druckfarben enthaltend die erfindungsgemäße Pigmentzubereitung sind inbesondere in Hinblick auf graphische Erzeugnisse der Werbebranche und bei hochwertigen Druckerzeugnissen von besonderer Bedeutung, da die fertigen Drucke aufgrund ihres Glanzes ästhetisch höchste Ansprüche erfüllen.

Zur Verbesserung der Druckgeschwindigkeiten und Fortdruckeigenschaften empfiehlt es sich, insbesondere beim Offsetdruck, Drucktücher mit einer glatten Oberfläche zu verwenden, wobei die modifizierte Oberfläche vorzugsweise aus Polyvinylchlorid, Polyurethan, Polyester oder Teflon bestehen und eine Oberflächenhärte von ca. 88 - 95° Shore aufweisen sollte. Die Verwendung eines solchen Drucktuchs in Kombination mit der erfindungsgemäßen pigmentierten Druckfarbe gewährleistet einen einwandfreien Farbübertrag.

Gegenstand der Erfindung sind somit auch Druckfarben, die bis zu 40 Gew.%, insbesondere 10 - 30 Gew.%, der erfindungsgemäßen Pigmentzubereitung enthalten.

Die erfindungsgemäße Pigmentzubereitung zeichnet sich durch ihren hohen Glanz aus und kann daher für die verschiedensten Zwecke eingesetzt werden. Neben der Verwendung in Druckfarben kann sie weiterhin in Kunststoffen, Farben, Lacken und aufgrund des guten Skin-Feelings auch in kosmetischen Zubereitungen Anwendung finden.

Gegenstand der Erfindung sind somit auch Formulierungen, die die erfindungsgemäße Pigmentzubereitung enthalten.

Die folgenden Beispiele sollen die Erfindung näher erläutern, ohne sie jedoch zu begrenzen.

### Beispiel 1

### I. Pigmentpräparation

1. Trockenmischung von Glanzpigment und sphärischen Teilchen
   300 g Iriodin 123 Hellglanzsatin (TiO₂-Glimmerpigment der Teilchengröße 5 - 20 µm der Fa. E. Merck, Darmstadt, BRD) werden mit 9 g Scotchlite S22 (Glashohlkugeln mit einer Teilchengröße von 70 µm der Fa. 3M Frankfurt, BRD) in einem 1,5-l-Kunststoffgefäß durch intensives Schütteln gemischt.
2. Herstellung der Suspension mit Natriumpolyphosphat
   100 g Drucköl L der Fa. Kast & Ehinger, Stuttgart, BRD und 100 g Natriumpolyphosphat der Fa. E. Merck, Darmstadt, BRD werden im Stahlgefäß des Dispermats mit Kugelmühleneinsatz (100 g Zirkonoxidkugeln mit einem Durchmesser von 1,25 - 1,6 mm) unter Wasserkühlung 2 - 3 Stunden bei 800 Umdrehungen dispergiert. Die Dispergierung ist beendet, sobald keine groben Partikel feststellbar sind ("Fingertest"). Die Suspension wird über ein Schnellsieb (E-D Sieb 100 µm) gegeben und zweimal mit je 25 g Drucköl L gewaschen.
3. Herstellung der Pigmentzubereitung
   300 g der Trockenmischung aus Schritt 1 und 20 g der Natriumpolyphosphatsuspension aus Schritt 2 werden intensiv miteinander gemischt.

### II. Herstellung einer Bogenoffsetdruckfarbe

700 g Bogenoffsetbindemittel (Gebr. Schmidt Bronzefirnis 11A10342; Viskosität gemessen an Laray Fallstabviskosimeter ca. 24 ± 2 Pa.s.) werden in einem 3-l-Rührgefäß mit Flügelrührer vorgelegt. Unter langsamen Rühren werden 320 g der Pigmentpräparation aus Schritt 3 portionsweise zugegeben. Dabei erwärmt sich die Mischung auf ca. 40 °C. Wenn die Bestandteile homogen verteilt sind, wird die Farbe luftdicht verschlossen. Nach 24 h Lagerung wird die Viskosität und Zügigkeit der Farbe bestimmt:
Eta* = 20 ± 4 Pa.s (20 °C)
Zügigkeit**: 120 ± 10 (30 °C)
* Viskositätsbestimmung mit einem Laray Fallstabviskosimeter (Lhomary S.A.) in Anlehnung an DIN 53222. Verwendete Gewichte: 800, 600, 400, 200 g
** Zügigkeitsmessung mit Tack-o-scope (Fa. Testprint B.V.); Walzen geschwindigkeit 100 m/min.

### Beispiel 2

### I. Pigmentzubereitung

720 g Iriodin 123 werden in einem 5 l Edelstahlgefäß vorgelegt und mit 21,6 g Scotchlite S22 (Glashohlkugeln der Firma 3M) versetzt und mit dem Dispermat intensiv gemischt. Anschließend wir eine Natriumpolyphosphatsuspension (hergestellt analog Beispiel 1) bestehend aus 49,4 g Natriumpolyphosphat und 24,7 g Drucköl L der Fa. Kast & Ehinger zugegeben und alles intensiv miteinander gemischt.

### II. Herstellung einer Offsetdruckfarbe

In einem 5 l Edelstahlgeföß mit Propellerrührer werden 1400 g Offsetbindemittel (Gebr. Schmitdt Bronzefirnis 11 A 1034) vorgelegt und unter Rühren portionsweise mit 680 g der Pigmentzubereitung aus I. versetzt. Wenn die Bestandteile homogen verteilt sind, wird die Farbe luftdicht verschlossen. Nach 24 h Lagerung wird analog Beispiel 1 II die Viskosität gemessen.
Eta = 4 ± 1 Pa.s (20°C)

### Beispiel 3 - Offsetdruckversuch

| | |
|---|---|
| Druckmaschine | MAN Roland 202 |
| | |
| Druckfarbe | Testfarbe aus Beispiel 1 |
| | |
| Papier | Kunstdruckpapier 115g/m² |
| | |
| Druckplatte | Ozasol P51 |
| | |
| Drucktuch | Spezialdrucktuch der Fa. Streb |
| | |
| Feuchtwasser | mit 10 % Alkohol |
| | |
| Druckparameter | Druck Platte-Gummituch 0,2 mm Beistelldruck 0,2 mm Farbgebung 110 % |

Das Endprodukt zeichnet sich durch seinen hohen Glanz aus. Ein Aufbauen der Glanzpigment-haltigen Druckfarbe auf der Drucktuchoberfläche wurde nicht beobachtet.

### Beispiel 4

### I. Pigmentpräparation

4.1. Trockenmischung von Glanzpigment und sphärischen Teilchen
   300 g Iriodin 123 Hellglanzsatin (TiO₂-Glimmerpigment der Teilchengröße 5 - 20 µm der Fa. E. Merck, Darmstadt, BRD) werden mit 9 g Scotchlite S22 (Glashohlkugeln mit einer Teilchengröße von 70 µm der Fa. 3M Frankfurt, BRD) in einem 1,5-l-Kunststoffgefäß durch intensives Schütteln gemischt.
4.2. Herstellung der Suspension mit Natriumpolyphosphat
   900 g Drucköl L der Fa. Kast & Ehinger, Stuttgart, BRD und 600 g Natriumpolyphosphat der Fa. E. Merck, Darmstadt, BRD werden im Stahlgefäß des Dispermats mit Kugelmühleneinsatz (2 kg Zirkonoxidkugeln mit einem Durchmesser von 1,2 -1,6 mm) unter Wasserkühlung 2 - 4 Stunden bei 2850-3000 Umdrehungen dispergiert. Die Dispergierung ist beendet, sobald keine groben Partikel feststellbar sind ("Fingertest"). Die Suspension wird über ein Schnellsieb (E-D Sieb 100 µm) gegeben.

### II. Herstellung einer Bogenoffsetdruckfarbe

700 g Bodenoffsetbindemittel (Gebr. Schmidt Bronzefirnis 11A 1034-1; Viskosität gemessen an Laray Fallstabviskosimeter η = 12 ± 2 Pa.s.) werden in einem 3-l-Rührgefäß mit Flügelrührer vorgelegt. Unter langsamen Rühren werden 300 g der Trockenmischung aus Schritt 4.1, 20 g der Natriumpolyphosphatsuspension aus Schritt 4.2 und 40 g Drucköl L der Fa. Kast & Ehinger nacheinander zugegeben. Dabei erwärmt sich die Mischung auf ca. 50 °C. Wenn die Bestandteile homogen verteilt sind, wird die Farbe luftdicht verschlossen. Nach 24 h Lagerung wird die Viskosität und Zügigkeit der Farbe bestimmt.
Eta* = 10 ± 2 Pa.s (25 °C)
Zügigkeit** = 120 ± 10 (32°C)
* Viskositätsbestimmungen mit einem Laray Fallstabviskosimeter (Lhomary S.A.) in Anlehnung an DIN 53222. Verwendete Gewichte: 800, 600, 400, 200 g
** Zügigkeitsmessung mit Tack-o-scope (Fa. Testprint B.V.): Walzengeschwindigkeit 100 m/min.

## Patentansprüche

1. Pigmentzubereitung im wesentlichen bestehend aus ein oder mehreren plättchenförmigen Glanzpigmenten, einem Phosphatderivat und sphärischen Teilchen mit einer Teilchengröße von 0,05 - 150 µm.

2. Pigmentzubereitung nach Anspruch 1, dadurch gekennzeichnet, daß sie bis 99,4 Gew.% an Glanzpigmenten, insbesondere Perlglanzpigmenten, enthält.

3. Pigmentzubereitung nach Anspruch 1, dadurch gekennzeichnet, daß sie das Phosphatderivat in Mengen von 0,1 - 5 Gew.% enthält.

4. Pigmentzubereitung nach Anspruch 1, dadurch gekennzeichnet, daß sie mindestens 0,5 Gew.% an sphärischen Teilchen bezogen auf das Glanzpigment enthält.

5. Pigmentzubereitung nach Anspruch 1, dadurch gekennzeichnet, daß die sphärischen Teilchen Glas-, Wachs- oder Polymerhohlkugeln oder SiO₂-Kugeln sind.

6. Pigmentzubereitung nach Anspruch 1, dadurch gekennzeichnet, daß sie zusätzlich bis 10 Gew.% einer flüssigen Komponente enthält.

7. Verwendung der Pigmentzubereitung nach Anspruch 1 in Druckfarben, Lacken, Farben, Kunststoffen und kosmetischen Zubereitungen.

8. Offset-Druckfarben enthaltend bis zu 40 Gew.% der Pigmentzubereitung nach Anspruch 1.

9. Formulierungen enthaltend die Pigmentzubereitung nach Anspruch 1.

## Claims

1. Pigment preparation consisting essentially of one or more platelet-shaped lustre pigments, a phosphate derivative and spherical particles having a particle size of 0.05 - 150 µm.

2. Pigment preparation according to Claim 1, characterized in that it contains up to 99.4% by weight of lustre pigments, especially pearl lustre pigments.

3. Pigment preparation according to Claim 1, characterized in that it contains the phosphate derivative in quantities of 0.1 - 5% by weight.

4. Pigment preparation according to Claim 1, characterized in that it contains at least 0.5% by weight of spherical particles, based on the lustre pigment.

5. Pigment preparation according to Claim 1, characterized in that the spherical particles are hollow glass, wax or polymer spheres or SiO₂ spheres.

6. Pigment preparation according to Claim 1, characterized in that it additionally contains up to 10% by weight of a liquid component.

7. Use of the pigment preparation according to Claim 1 in printing inks, coatings, paints, plastics and cosmetic preparations.

8. Offset printing inks containing up to 40% by weight of the pigment preparation according to Claim 1.

9. Formulations comprising the pigment preparation according to Claim 1.

## Revendications

1. Préparation pigmentaire essentiellement constituée d'un ou plusieurs pigments brillants lamellaires, d'un dérivé de phosphate et de particules sphériques ayant une dimension comprise entre 0,05 et 150 µm.

2. Préparation pigmentaire selon la revendication 1, caractérisée en ce qu'elle contient jusqu'à 99,4 % en poids de pigments brillants, en particulier de pigments nacrés.

3. Préparation pigmentaire selon la revendication 1, caractérisée en ce qu'elle contient 0,1 à 5 % en poids du dérivé de phosphate.

4. Préparation pigmentaire selon la revendication 1, caractérisée en ce qu'elle contient au moins 0,5 % en poids de particules sphériques rapporté aux pigments brillants.

5. Préparation pigmentaire selon la revendication 1, caractérisée en ce que les particules sphériques sont des billes creuses en verre, en cire ou en polymère ou des billes de SiO₂.

6. Préparation pigmentaire selon la revendication 1, caractérisée en ce qu'elle contient en plus jusqu'à 10 % en poids d'un composant liquide.

7. Utilisation d'une préparation pigmentaire selon la revendication 1 dans les encres d'imprimerie, les vernis, les peintures, les matières plastiques et les préparations cosmétiques.

8. Encres pour l'impression offset contenant jusqu'à 40 % en poids de la préparation pigmentaire selon la revendication 1.

9. Formulations contenant la préparation pigmentaire selon la revendication 1.
